# EUROPEAN PATENT APPLICATION

(11) **EP 4 548 973 A2**
(43) Date of publication of application: **07.05.2025**
(21) Application number: 25163026.5
(22) Date of filing: 27.05.2020
(51) Int. Cl.: A61P 27/02

(54) **COMPOSITIONS AND METHODS FOR TREATING RETINOPATHY**

(30) Priority: 28.05.2019 US 201962853179 P
(62) Divisional of application: 20743857.3
(71) Applicant: Elgan Pharma Ltd., 1603611 Nazareth (IL)
(72) Inventor: OLSHANSKY, Michal, 6233112 Tel Aviv (IL); OSTROVSKY, Elena, 7523405 Rishon-LeZion (IL); ZELDIS, Stav, 3057021 Binyamina-Giv'at Ada (IL)
(74) Representative: Dilg, Haeusler, Schindelmann Patentanwaltsgesellschaft mbH

(57) **Abstract**

A pharmaceutical composition including insulin, Docosahexaenoic acid (DHA) and coenzyme Q10 and methods of manufacturing and using the composition are provided.

## Description

### REPLATED APPLICATION

This application claims the benefit of priority of U.S. Provisional Patent Application No. 62/853,179 filed on May 28, 2019, the contents of which are incorporated herein by reference in their entirety.

### BACKGROUND

The present invention relates to a composition-of-matter for treating retinopathy. Embodiments of the present invention relate to a nanoemulsion including Insulin and/or IGF for treating retinopathy of prematurity (ROP).

Birth is considered premature, or preterm, when it occurs before the 37th week of pregnancy. The final weeks in the womb are crucial for healthy weight gain and for the full development of various vital organs.

In humans, the retina develops in-utero where tissue oxygen is low. Vascular precursor cells are laid from 12 to 21 weeks gestational age creating a scaffold for future vessel development. Retinal angiogenesis begins at approximately 16 weeks gestational age, with new vessels budding from existing vessels. The metabolic demands of the developing retina exceed the oxygen supplied by the choroidal circulation resulting in "physiologic hypoxia," that stimulates angiogenesis.

Retinopathy of prematurity (ROP) is a developmental vascular disorder characterized by abnormal growth of retinal blood vessels in the incompletely vascularized retina. ROP mostly occurs in extremely low gestational age neonates (ELGANs) who are 1250 g, or under 28 weeks gestation at birth and is the most common cause of visual impairment and blindness in children.

Current treatment options including laser photocoagulation and intravitreal injection of vascular endothelial growth factor (VEGF) antibodies have proven to be useful in severe late ROP. However, laser photocoagulation destroys major parts of the retina and is a difficult and complicated procedure to perform in young infants while intravitreal injection of VEGF antibodies may cause a systemic suppression of vascular growth affecting other organs.

There is thus a need for, and it would be highly advantageous to have a retinopathy treatment approach devoid of the above limitations.

### SUMMARY

According to one aspect of the present invention there is provided a pharmaceutical composition comprising insulin, Docosahexaenoic acid (DHA) and coenzyme Q10.

According to another aspect of the present invention there is provided a method of treating retinopathy in preterm infants comprising administering a pharmaceutical composition including insulin, Docosahexaenoic acid (DHA) and coenzyme Q10 to an eye of a preterm infant thereby treating retinopathy in preterm infants.

According to another aspect of the present invention there is provided a method of preventing or reducing severity of retinopathy in preterm infants comprising administering a pharmaceutical composition including insulin, Docosahexaenoic acid (DHA) and coenzyme Q10 to an eye of a preterm infant thereby preventing or reducing severity of retinopathy in preterm infants.

According to another aspect of the present invention there is provided a method of reducing retinal hemorrhages in preterm infants comprising administering a pharmaceutical composition including insulin, Docosahexaenoic acid (DHA) and coenzyme Q10 to an eye of a preterm infant thereby reducing retinal hemorrhages in preterm infants.

According to another aspect of the present invention there is provided a method of reducing retinal hemorrhages in subjects experiencing retinopathy comprising administering a pharmaceutical composition including insulin, Docosahexaenoic acid (DHA) and coenzyme Q10 to an eye of a subject thereby reducing retinal hemorrhages in subject eye.

According to another aspect of the present invention there is provided a method of reducing retinal neovascularization in subjects experiencing retinopathy comprising administering a pharmaceutical composition including insulin, Docosahexaenoic acid (DHA) and coenzyme Q10 to an eye of a subject thereby reducing retinal neovascularization in subject eye

According to another aspect of the present invention there is provided a method of increasing retinal vascular coverage in preterm infants comprising administering a pharmaceutical composition including insulin, Docosahexaenoic acid (DHA) and coenzyme Q10 to an eye of a preterm infant thereby increasing retinal vascular coverage in preterm infants (reducing avascular retinal areas).

According to another aspect of the present invention there is provided a method of reducing retinal inflammation in preterm infants comprising administering a pharmaceutical composition including insulin, Docosahexaenoic acid (DHA) and coenzyme Q10 to an eye of a preterm infant thereby reducing retinal inflammation in preterm infants.

According to another aspect of the present invention there is provided a method of reducing retinal oxidative stress in preterm infants comprising administering a pharmaceutical composition including insulin, Docosahexaenoic acid (DHA) and coenzyme Q10 to an eye of a preterm infant thereby reducing retinal oxidative stress in preterm infants.

According to another aspect of the present invention there is provided a method of improving retinal layer development in preterm infants comprising administering a pharmaceutical composition including insulin, Docosahexaenoic acid (DHA) and coenzyme Q10 to an eye of a preterm infant thereby improving retinal layer development in preterm infants.

According to another aspect of the present invention there is provided a method of reducing vision impairment (incidence or severity) in preterm infants comprising administering a pharmaceutical composition including insulin, Docosahexaenoic acid (DHA) and coenzyme Q10 to an eye of a preterm infant thereby reducing vision impairment in preterm infants.

According to another aspect of the present invention there is provided a method of increasing visual field in preterm infants comprising administering a pharmaceutical composition including insulin, Docosahexaenoic acid (DHA) and coenzyme Q10 to an eye of a preterm infant thereby increasing visual field in preterm infants.

According to another aspect of the present invention there is provided a method of formulating a pharmaceutical composition for topical treatment of retinopathy comprising: (a) generating an oil-in-water nanoemulsion including Docosahexaenoic acid (DHA) and Coenzyme Q10 in the oil phase; and (b) conjugating Insulin or IGF-1 to nanodroplets of the nanoemulsion using an amine coupling reaction.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE SEVERAL VIEWS OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 schematically illustrates the present composition.
FIGs. 2A-B are graphs of in-vivo fundoscopy results representing total retinal hemorrhages (Figure 2A) and total severe hemorrhages (Figure 2B).
FIGs. 3A-C are images of in Vivo fundoscopy results of normoxia animals (Figure 3A), untreated hypoxic animals (Figure 3B) and treated animals (Figure 3C).
FIGs. 4A-B are graphs showing the effect of treatment on Neovascularization at days 14 and 18.
FIGs. 5A-D illustrate Isolectin-B4 staining of P14 for the insulin treated group (Figure 5A), the IGF-1 treated group (Figure 5B), the untreated group (Figure 5C) and the normoxia (healthy) animals (Figure 5D).
FIGs. 6A-B are graphs showing the avascular area of the insulin treated, IGF-1 treated, untreated and normoxia groups.
FIGs. 7A-D are images showing isolectin-B4 staining of P14, for the insulin (Figure 7), IGF-1 (Figure 7B), untreated (Figure 7C) and normoxia (Figure 7D) groups. ROI (green), vessels covered area (blue), vessels skeleton (red) and branching points (white) are marked.
FIG. 8 is a chromatogram of the coupling reaction at certain time point with all reaction constituents, e.g. reactants (rh-Insulin and DHA), an intermediate (DHA-EDC intermediate) and resulting product (Insulin-DHA conjugate).
FIG. 9 is a chromatogram of Insulin-DHA conjugate extracted from the lyophilized emulsion (finished product formulation).
FIG. 10 is a chromatogram showing the peaks of the constituents of the lyophilized emulsion (finished product formulation).
FIGs. 11A-C are graphs illustrating the results of the automated analysis of H&E retinal layers thickness using Wimretina software (Figure 11A), the results of biomarker PGE2 levels analysis performed in the study, comparing the different study groups (Figure 11B) and the results of biomarker 8-iso-PGF2a levels analysis performed in the study, comparing the different study groups (Figure 11C).
FIGs. 12-13 are chromatograms showing the peaks of the constituents of the formulation containing free Insulin, DHA and Coenzyme Q10.
FIGs. 14A-E and 15A-E are cryo transmitting electron micrographs (TEM) of the composition of the present invention.

### DETAILED DESCRIPTION

The present invention is of a composition-of-matter which can be used to treat retinopathy. Specifically, the present invention can be used to treat ROP via local administration of nanoemulsion including Insulin or IGF.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Although treatment options for ROP exist, such options are limited by complexity of administration, side effects and possible damage to eye tissue.

The present inventor postulated that effective treatment of ROP should prevent the toxic post-birth influences (e.g., oxygen excess) and provide missing intrauterine factors (insulin and insulin growth factor 1) that can promote physiological vasculature development while minimizing systemic exposure to these factors.

While reducing the present invention to practice, the present inventor formulated compositions that can promote physiological eye vasculature development and reduce intraocular toxicity thereby enabling treatment of retinal disorders such as retinopathy. As is further described in the Examples section that follows, the present compositions were effective in stimulating healthy vessel growth and preventing and reducing retinal hemorrhages and pathological blood vessel growth (neovascularization) caused by the Oxygen-Induced model in a rat.

The phrase "promoting physiological vascular development" refers to increasing the flow or passage of oxygen from the optic nerve to the periphery of the eye.

The term "retinopathy" refers to any damage to the retina which may cause vision impairment. This can include, for example, a pathology that slows or stops the growth of physiological vasculature (vaso-obliterative or constrictive stage, e.g., phase I of ROP) and the abnormal (aberrant) pathological blood vessels that are formed in response to tissue hypoxia and ischemia. Retinopathy can be a result of external factors such as radiation or head trauma or a manifestation of a systemic disease such as diabetes or hypertension. Retinopathy can also be caused by vascular inflammation and medications (e.g., diabetes medications such as Exenatide, Liraglutide, and Pramlintide).

Thus, according to one aspect of the present invention there is provided a composition-of-matter including a therapeutically effective amount of insulin and/or IGF-1, Docosahexaenoic acid (DHA) and coenzyme Q10 as active ingredients.

As is further described herein the insulin and/or IGF-1 promote promoting physiological vascular development while the DHA reduces the inflammatory response and coenzyme Q10 reduces the oxidative stress signaling.

The term "therapeutically effective amount" or "pharmaceutically effective amount" denotes that dose of an active ingredient or a composition comprising the active ingredient that will provide the therapeutic effect for which the active ingredient is indicated.

The dose of each active ingredient in the present pharmaceutical composition can depend on many factors including the subject being treated, the stage of retinopathy (e.g., ROP) and the route of administration (topical or intraocular).

In the case of ROP, progression can be determined via somatic effects (e.g., vessel density and coverage), extent and/or progression of vascularization or quality of retinal layers development.

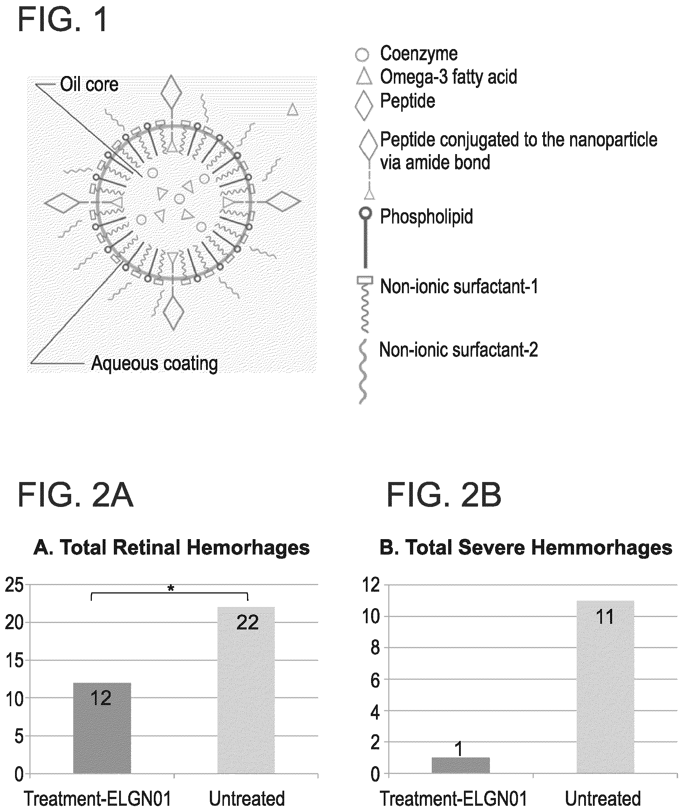

The composition-of-matter can be formulated as a water-in-oil nanoemulsion having nanodroplets that include the Docosahexaenoic acid (DHA) and coenzyme Q10 and are conjugated to insulin and/or IGF (via, for example, an amide bond). Figure 1 is a schematic illustration of the present composition-of-matter showing nanodroplets conjugated to Insulin or IGF 12 and containing DHA 12 and coenzyme Q10 14.

The Examples section that follows describes one approach for formulating the present composition-of-matter.

The composition-of-matter can be stored in a lyophilized state and reconstituted with water or saline for example for use or stored as a ready for use pharmaceutical composition.

The composition-of-matter can be a part of a pharmaceutical composition that includes a carrier formulated for topical or intra-ocular delivery.

Topical formulations of the present pharmaceutical composition can include a carrier such as Medium-chain triglycerides (MCT), long-chain triglycerides oils such as castor oil, synthetic and semi-synthetic oils such as Mineral Oil and unsaturated fatty acids such as oleic acid.

Intra-ocular formulations of the present pharmaceutical composition can formulated as a microemulsion and/or include a carrier such as liposomes, nanospheres, micelles and nanocapsules.

Intraocular formulation can be formulated for slow or delayed release of the active ingredients using excipients which form inclusion complexes with active ingredients such as chelating agents, surfactants, and cyclodextrins.

The pharmaceutical composition can also include:
(i) Carbohydrates (as stabilizers, lubricants, or cryoprotectants) including, but not limited to monosaccharides (e.g. glucose, maltose), disaccharides (e.g. trehalose), oligosaccharides (dextrins (e.g. Maltodextrin), cyclodextrins (e.g. Hydroxypropyl-beta-cyclodextrin (HPbCD), polysaccharides (e.g. dextran).
(ii) Emulsifiers including, but not limited to nonionic surfactants of natural origin (e.g. lecithin, egg yolk phospholipids) and synthetic origin (e.g. Tyloxapol) and ionic surfactants (e.g. cetalkonium chloride).
(iii) Thickening agents including, but not limited to hydrophilic polymers (e.g. Polyvinyl alcohol) or cellulose derivatives (e.g. hydroxy propyl methyl cellulose (HPMC).
(iv) A bioadhesive such as polyaminoacids (e.g., gelatin, human albumin) and polysaccharides such as cellulose derivatives (e.g. hydroxy propyl methyl cellulose (HPMC) and hydroxypropyl cellulose (HPC), hyaluronic acids
(v) Gelling agent such as alginate and poly acrylates, can be added to the pharmaceutical composition to increase the residence time of the active ingredients on the cornea.

According to embodiments of the present invention, the concentration of insulin in the pharmaceutical composition can be 0.001U to 20U per ml while the concentration of IGF can be 0.001U to 20U per ml.

According to embodiments of the present invention, the concentration of DHA in the pharmaceutical composition can be 1-4 mg/ml.

According to embodiments of the present invention, the concentration of coenzyme Q10 in the pharmaceutical composition can be 1-3 mg/ml.

Table 1 below describes a topical formulation of the present composition.

**Table 1 - Topical Formulation**

| **Ingredient** | **Quantity per vial** | |
|---|---|---|
| | **ELGN01** | **ELGN02** |
| Recombinant Human Insulin (rh-Insulin) | 0.067 IU | - |
| Insulin-Like Growth Factor 1 (IGF-1) | - | 2 IU |
| Cis-4,7,10,13,16,19- Docosahexaenoic acid (DHA) | 2mg | 2mg |
| Coenzyme Q10 (CoQ10) | 1mg | 1mg |
| Medium-chain triglycerides (MCT) | 1mg | 1mg |
| Tyloxapol | 0.5mg | 0.5mg |
| Lipoid E 80 | 0.5mg | 0.5mg |
| Polyvinyl alcohol (PVA) | 1mg | 1mg |
| Hydroxypropyl-beta-cyclodextrin (HPbCD) | 2mg | 2mg |

The present formulation can be modified to be free of MCT and include DHA in two forms - as free acid and as ethyl ester. These two forms of DHA replaces MCT in the droplet core. Two separate emulsions are produced and combined at the last production step. One emulsion contains the droplets that include the DHA free acid to which Insulin is conjugated. The second emulsion contains droplets in which Q10 is incorporated to the DHA ethyl ester core. Table 2 below lists the ingredients of this embodiment of the present formulation in an injectable form.

**Table 2**

| **Ingredient** | **Function** | **Quantity per 1ml** |
|---|---|---|
| Recombinant Human Insulin (rh-Insulin) | Active ingredient | 2 IU (0.07mg) |
| Cis-4,7,10,13,16,19- Docosahexaenoic acid* (DHA) | Excipient | 1.87mg |
| Coenzyme Q10 (CoQ10) | Excipient | 1.0mg |
| Tyloxapol | Surfactant | 0.5mg |
| Lipoid E 80 | Surfactant | 0.5mg |
| Polyvinyl alcohol (PVA) | Surfactant | 1.0mg |
| Hydroxypropyl-beta-cyclodextrin (HPbCD) | Stabilizing agent | 40mg |
| Sodium hydroxide*** | adjustment pH value | - |
| Hydrochloric acid** | adjustment pH value | - |
| Water for injection | Solvent | Up to 1ml |

| | | |
|---|---|---|
| *Contains about 0.4mg/ml DHA free acid and about 1.6mg/ml DHA ethyl ester (corresponds to 1.47mg/ml DHA free acid) **Sodium hydroxide or hydrochloric acid are used for adjusting the pH value and not included in the sum. | | |

An intraocular formulation of the present composition is described in Table 3 below.

**Table 3 - Intraocular formulation**

| **Ingredient** | **Quantity per vial** |
|---|---|
| | **ELGN03** |
| Recombinant Human Insulin (rh-Insulin) | 0.005-0.1 IU |
| Cis-4,7,10,13,16,19- Docosahexaenoic acid (DHA) | 0.2-0.5mg |
| Coenzyme Q10 (CoQ10) | 0.1mg |
| Medium-chain triglycerides (MCT) | 0.11mg |
| Tyloxapol | 0.05mg |
| Lipoid E 80 | 0.05mg |
| Polyvinyl alcohol (PVA) | 0.1mg |
| Hydroxypropyl-beta-cyclodextrin (HPbCD) | 0.2mg |

In order to enhance the efficacy of the present composition, an approach for manufacturing a nanoemulsion having nanodroplets encapsulating the (DHA) and Coenzyme Q10 and conjugated to Insulin or IGF was developed.

Thus, according to another aspect of the present invention there is provided a method of formulating a pharmaceutical composition for topical treatment of retinopathy. The pharmaceutical composition is manufactured by generating an oil-in-water nanoemulsion including Docosahexaenoic acid (DHA) and Coenzyme Q10 in the oil phase and conjugating Insulin or IGF-1 to nanodroplets of the nanoemulsion using an amine coupling reaction.

Following nanoemulsion generation, the nanodroplets can be purified or concentrated using, but not limited to, column chromatography, tangential flow filtration (TFF), dialysis. Stabilizing agent such as, but not limited to, cyclodextrin, dextrin, mono-or disaccharide can be added.

The formulation can then be lyophilized for storage and subsequent reconstitution with saline or water prior to use.

The Examples section that follows provide a more detailed description of the present formulation approach.

As is mentioned hereinabove, the present composition can be used to treat retinopathy and in particular retinopathy of prematurity (ROP).

Thus, according to another aspect of the present invention there is provided a method of treating retinopathy in a subject in need such as a preterm infant. The method is effected by administering the pharmaceutical composition of the present invention to an eye of the subject in need. Such administration can be topical or intraocular.

As used herein the phrase "subject in need thereof" refers to a human or non-human mammal. The human or non-human mammal (cats, dogs, cattle, sheep, pigs, goats and equines) at any age (e.g., infant such as term or preterm infant, adult or old) or sex. A human subject can be a preterm infant born at a gestational age of 24 to 33 weeks. The human subject can also be a low birth weight infant weighing 500 to 1650 gm at birth.

A topical formulation (eyedrops) of the present composition can be administered to a preterm infant anytime between birth and 6 months of age once or several times daily over a period of 180 days at a dose of 10 micro liter to 100 microliter. An intraocular formulation of the present composition can be administered to a preterm infant anytime between birth and 6 months of age once every several weeks over a period of 180 days, as clinically needed at a dose of five to 30 micro liters per injection.

As used herein the term "about" refers to ± 10 %.

Additional objects, advantages, and novel features of the present invention will become apparent to one ordinarily skilled in the art upon examination of the following examples, which are not intended to be limiting.

### EXAMPLES

Reference is now made to the following examples, which together with the above descriptions, illustrate the invention in a non-limiting fashion.

### EXAMPLE 1

### Nanoemulsion Formulation

The following example demonstrates manufacturing of the present composition-of-matter formulated as a lyophilized powder suitable for reconstitution as an oil-in-water nanoemulsion.

Table 4 below lists the ingredients used in the manufacturing process of the composition-of-matter formulation.

**Table 4 - formulation components**

| Ingredient | Function |
|---|---|
| Recombinant Human Insulin (rh-Insulin) | Active substance |
| Or | |
| Insulin-Like Growth Factor 1 (IGF-1)¹ | |
| | |
| Cis-4,7,10,13,16,19- Docosahexaenoic acid (DHA) | Active substance |
| Coenzyme Q10 synthetic (Q10) | Active substance |
| Medium-chain triglycerides (MCT) | Oil phase |
| Tyloxapol | Nonionic surfactant |
| Lipoid E 80 | Amphiphilic surfactant |
| Polyvinyl alcohol (PVA) | Nonionic surfactant |
| 0.5N NaOH¹ | Reagent, pH adjustment |
| Water (DDW)² | Aqueous phase |
| Acetone² | Solvent |
| Ethanol ² | Solvent |
| 1-ethyl-3-(3-dimethylaminopropyl)carbodiimide hydrochloride (EDC)^{2, 3} | Coupling Reagent |
| Phosphate buffered saline (PBS), pH 7.2 | Buffer |
| Sodium carbonate buffer | Reagent, pH adjustment |
| 2-Hydroxypropyl-β-cyclodextrin (HPbCD) | Stabilizer, cryoprotectant |

| | |
|---|---|
| ¹ Example 1 describes certain manufacturing process of insulin-containing formulation. ² Removed during the process ³ Crosslinking reagent used in amine coupling reaction and removed afterwards | |

Formulation of nanodroplets was achieved using solvent displacement method. 100 mg of DHA, 50 mg CoQ10, 25 mg Tyloxapol and 50 mg MCT were dissolved in 9 ml of Acetone and 25mg of Lipoid E80 were dissolved in 1ml of Ethanol. Resulting solutions were combined, mixed at 900 rpm for 30 minutes at room temperature and drop-added to 20 ml of PVA aqueous solution, 0.1%w/v, continuously stirred at 900 rpm for additional 15 minutes. Afterwards, the organic solvents were completely removed at room temperature under reduced pressure (50 mBar) using a laboratory rotary evaporator and the obtained emulsion was subjected to amine coupling reaction after preliminary pH adjustment to 7.4 with 0.5M NaOH.

1.3 µmol of EDC prepared in 0.5ml phosphate buffered saline (pH 7.2) was added to the resulting emulsion, the mixture was incubated at room temperature for 15 minutes and then the pH was adjusted to 8.3±0.2 using a sodium carbonate buffer. 0.5ml of 0.1µmol/ml rh-Insulin solution in phosphate buffered saline (pH 7.2) was added to 19.5ml of emulsion. The reaction mixture was set to stir for 12 hours at room temperature. Then, the reaction mixture was loaded onto a gravity-flow PD-10 gel filtration column (Sephadex G-25), using water as eluent, for separation of the nano-droplets from the smaller size particles (e.g. EDC, free active substance molecules). Excess of eluent (water) was removed from the nano-droplets fraction under reduced pressure (50 mBar) and 37°C using a laboratory rotary evaporator. Then, the emulsion was mixed with 2-Hydroxypropyl-β-cyclodextrinto a final concentration 2% w/v, filtered through 0.45µm PES (Polyethersulfone) membrane, dispensed to vials and lyophilized. Content of active components in 1ml of the reconstituted solution was 0.67U rh-Insulin, 2mg DHA and 1mg CoQ10.

### EXAMPLE 2

### Study 1

An ophthalmic formulation (ELGN01 consisting of Insulin DHA and Coq10) of the composition-of-matter described in Example 1 was tested on oxygen-Induced Retinopathy model in rats.

### Procedure

A single rat dam, with a litter of 18 pups was divided into two groups: Group A - ELGN01 (9), Group B - untreated (9, in the oxygen chamber without treatment). A single dam with 3 pups placed in normoxia conditions was used as an additional control.

Treatment was initiated at day 5-14 or 18 (according to sacrifice day), at first via administration under the eyelid with syringe (topical, not damaging the ocular surface) and then with ocular drops after eye opening.

The oxygen regimen was as follows: day 0-14 of life, 24-hours cycles of hyperoxia (50%), then Hypoxia (12%) for 24 hours.

The study of group 1 was terminated at day 14 (P14) of life and of group 2 at day 18 (P18) of life and a fundoscopy assessment was done at day 17 by an ophthalmologist, samples were then histologically and immune-histologically assessed.

### Results

### In-Vivo Fundoscopy results

In the treated group, there was a total of 12 retinal hemorrhages observed, compared to 22 in the untreated group animals (Treatment ELGN01 significance p=0.04).

**Table 5 - fundoscopy in-vivo results - retinal hemorrhages per group**

| | **Treatment- ELGN01** | **Untreated** | **Normoxia** |
|---|---|---|---|
| Number of all retinal hemorrhages | 12* | 22 | 0 |
| Number of severe retinal hemorrhages (Medium-Large) | 1 | 11 | 0 |
| Average number of retinal hemorrhages per eye | 1.4 | 2.75 | 0 |

Data are mean ± SD; (†) p<.1, (*) p< 0.05, (**) p< 0.01 by T-Test compared to control- untreated group.

Total retinal damage and total severe hemorrhages in the treated and untreated groups are shown in the graphs of Figures 2A-B.

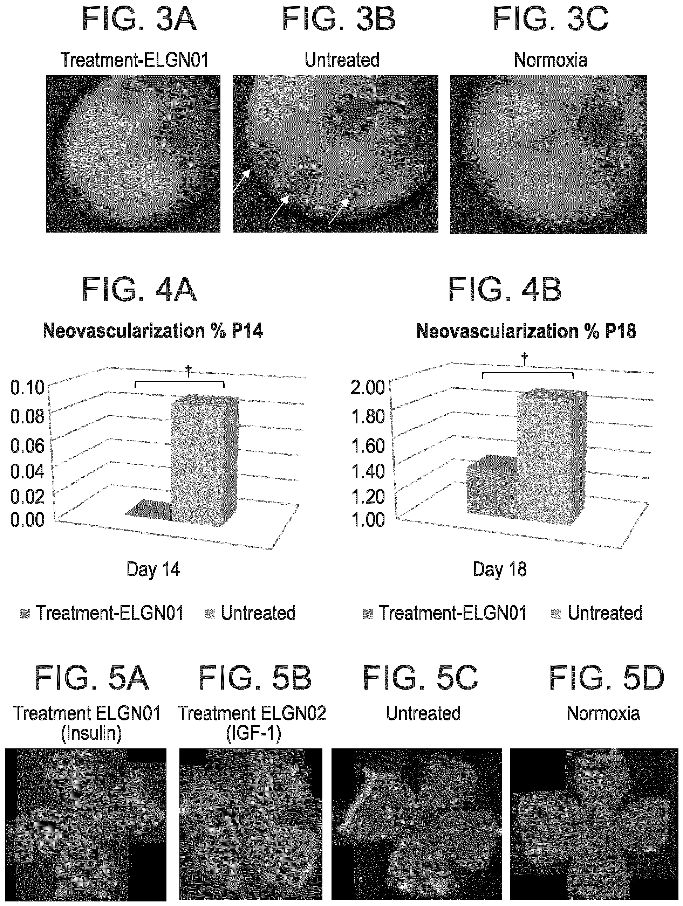

Figures 3A-C are images of the retina of normoxia, hypoxia (treated and untreated). Normoxia animals present intact vessels in the retina, no hemorrhages or ablation. Untreated hypoxic animals present retinal bleedings (arrows). Treated animals show reduced damage.

### Neovascular area

The effect on neovascularization is presented in Figures 4A-B. Neovascularization (NV) was higher at P18 and corresponded to end of phase II of human disease. P14 corresponded to end of phase I of disease (progression stage). In both time points the treatment group showed significantly less NV than untreated animals. In P14, ELGN01 Treatment group showed 0% neovascularization compared to 0.09% in the untreated group (T-Test comparison p=0.07). In P18, ELGN01 Treatment group showed 40% less neovascularization on average compared to the untreated group (Treatment ELGN01 1.35%, untreated 1.89%, T-test comparison p=0.07, a 28% treatment effect).

### Retinal layers

Whole eyes embedded in paraffin were sectioned and stained with haematoxylin & eosin. Four sections from different locations were collected on one slide. H&E stainings were imaged with light microscope with 10x objective (4x at some locations).

Representative stainings from the untreated OIR group show disorganization of retinal layers, thickening of the ganglion cell layer as a result of the OIR damage. A total of 8 samples were available per group - 4 sections per eye, 2 eyes per treatment group (from different animals).

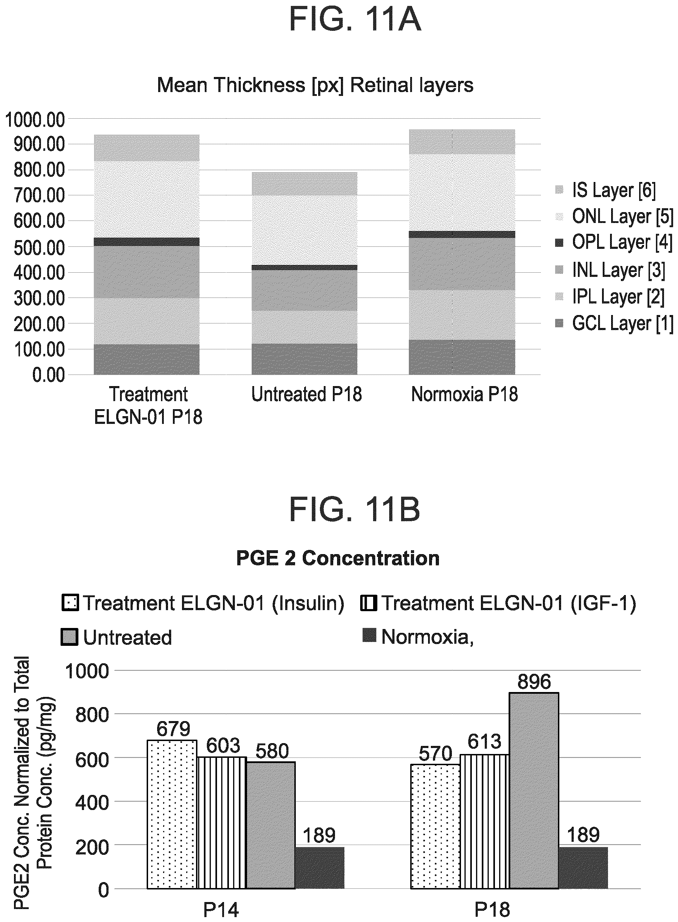

To assess retinal layers integrity, deidentified H&E-stained images were uploaded into the Wimasis Software Image. Each retina was analyzed in a masked manner, identifying and measuring the size of each retinal layer- RGCL - retinal ganglion cell layer, IPL - inner plexiform layer, INL - inner nuclear layer, OPL - outer plexiform layer, ONL - outer nuclear layer, RPE - retinal pigment epithelium, CHO - choroid. Figure 11A shows the mean thickness of the retinal layers.

**Table 6 - Mean Thickness [px] of retinal layers**

| | GCL Layer [1] | IPL Layer [2] | INL Layer [3] | OPL Layer [4] | ONL Layer [5] | IS Layer [6] |
|---|---|---|---|---|---|---|
| Treatment ELGN01 P18 | 118.80 | 180.23 | 203.73 | 32.02 | 298.71 | 103.95 |
| Untreated P18 | 121.91 | 128.10 | 157.90 | 21.33 | 270.20 | 90.71 |
| Normoxia P18 | 137.04 | 192.42 | 203.61 | 28.32 | 298.38 | 97.35 |

### EXAMPLE 3

### Study 2

Ophthalmic formulations based on the composition-of-matter described in Example 1 (ELGN01 consisting of Insulin DHA and Coq10, and ELGN02 consisting IGF-01 and same) were tested on oxygen-Induced Retinopathy model in rats.

### Procedure

Two rat dams, each including 18 pups were divided into three treatment groups: Group A ELGN01 (12), Group B ELGN02(12), Group C untreated (12). Group D Normoxia was analyzed as control.

Treatment was initiated at day 5, animals were administered treatment until day 14 or day 18 (according to sacrifice day), at first under the eyelid with syringe (topical, not damaging the ocular surface), then with ocular drops after eye opening.

The oxygen regimen was as follows: In the first 4 days, 8 intermittent hypoxia events of 3 reductions to 12% during 30 minutes event and rest of time 50% hyperoxia were carried out. Day 5-14 of life, 24-hours cycles of hyperoxia (50%), then Hypoxia (12%) for 24 hours.

The study of Group 1 was terminated at day 14 of life and a fundoscopy assessment was done at day 17 by an ophthalmologist, samples were then histologically and immune-histologically assessed.

### Results

### Isolectin Staining

Samples were flat mounted and retinas were stained with Isolectin GS-IB4. Avascular areas (AVAs) were manually quantified by an independent expert using the images of Isolectin stained retinas

Figures 5A-D illustrate staining of the insulin and IGF treated groups, the untreated group and the normoxia group. In the insulin and IGF-1 treated groups (Figures 5A-B) a minimal avascular area with complete central blood vessels is observed. In the untreated group (Figure 5C) large avascular areas are observed (arrows). In the normoxia group (Figure 5D) full coverage of the blood vessels is observed.

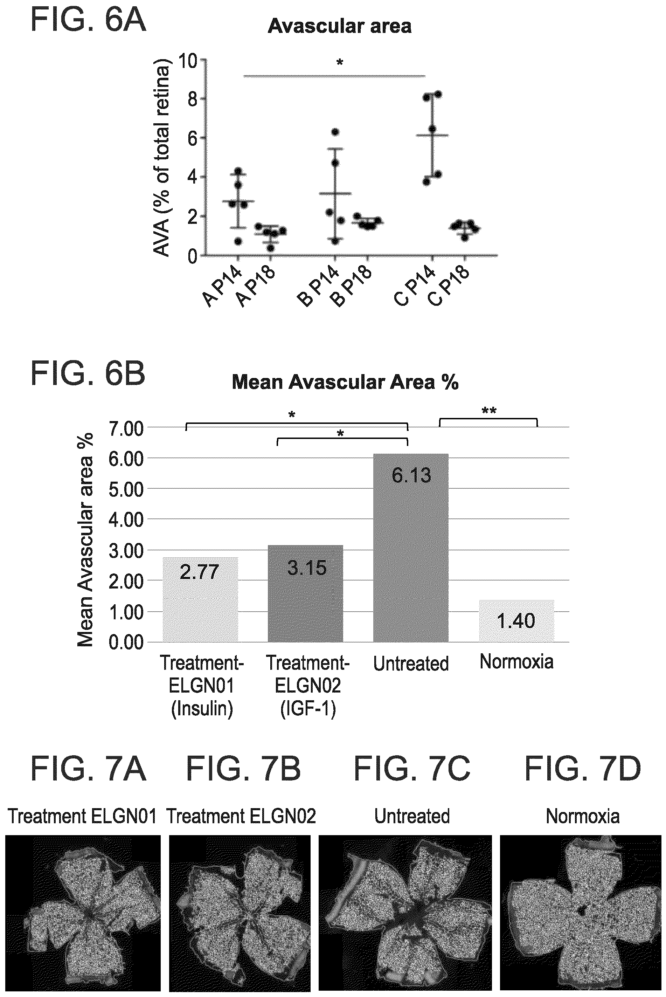

Figures 6A-B are graphs representing AVA. In P14, both treatment groups demonstrated 50% reduction of AVA in treatment groups, compared to control (2.77% Treatment ELGN01, 3.15% Treatment ELGN02, 6.13% untreated percentage of avascular area). The normoxia group presented 1.4% of avascular area. When using T-Test to compare the treatment groups to untreated animals, Treatment- ELGN01vs. untreated had a statistically significant difference (p= 0.01), as well as Treatment- ELGN02vs. untreated (p=0.03).

Each retina was analyzed in a masked manner for vascular density (%, calculated by dividing the number of pixels of the vessels by the total number of pixels of the region of interest), total vascular area, number of branching points (where two or more segments converge), number of segments (number of individual vessel segments), and mean segment length.

In P14, both treatment groups were superior to untreated animals in numerous characteristics; Treatment ELGN01 had significantly higher vessels density (%) compared to untreated (p=0.051), as well as Treatment ELGN02 (p=0.032) indicating better growth and development of the blood vessels in the retina, as well as less avascular areas. Treatment groups also showed larger vascular area compared to untreated group- treatment ELGN01 (p=0.073) and treatment ELGN02 (p=0.014). In addition, Treatment A - ELGN01 had significantly higher mean segment length (p=0.037) compared to untreated animals, indicating better continuity of the blood vessels.

**Table 7 - quantitation of retinal vasculature at P14**

| | **Treatment - ELGN01** | **Treatment - ELGN02** | **Untreated** | **Normoxia** |
|---|---|---|---|---|
| Vessels density (%) | 57.04 ± 2.15† | 57.3 ± 1.5* | 54.1 ± 2.82 | 65.47 ± 0.31 |
| Total vascular area (in 1,000,000 px units) | 3.826 ± 0.23† | 3.978 ± 0.2* | 3.571 ± 0.26 | 4.28 ± 0.05 |
| Total Branching Points | 6065 ± 1039 | 6958 ± 407 | 6700 ± 555 | 9710 ± 97 |
| No. Segments | 6464 ± 1134 | 7398 ± 414 | 7252 ± 619 | 10,034 ± 111 |

| | | | | |
|---|---|---|---|---|
| Data are mean ± SD; (†) p<.1 , (*) p< 0.05, (**) p< 0.01 by T-Test compared to control-untreated group. N=5 per group | | | | |

Figures 7A-D are images of isolectin-B4 staining of P14, per treatment group. ROI (green), vessels covered area (blue), vessels skeleton (red) and branching points (white) are marked.

As is outlined in Table 8 below, in P18 treatment ELGN01 had significantly higher vessels density (%) compared to untreated (p=0.007). Treatment ELGN02 showed a non-significant trend. Vessels density % reflects the amount of the retina that is vascularized compared to non-vascularized area. Higher vessels density without neovascularization indicates better growth and development of the blood vessels in the retina, as well as less avascular areas. Treatment groups also showed a trend for larger vascular area compared to untreated group. Treatment ELGN01 showed higher number of branching points compared to the untreated group (p=0.02). In addition, Treatment ELGN01 showed a larger amount of blood vessels compared to untreated group (p=0.04).

**Table 8 - quantitation of retinal vasculature at P18**

| | **Treatment-ELGN01** | **Treatment - ELGN02** | **Untreated** | **Normoxia** |
|---|---|---|---|---|
| Vessels density (%) | 62.24 ± 1.15 ** | 60.38 ± 1.67 | 59.46 ± 1.6 | 65.83 ± 0.87 |
| Total vascular area (in 1,000,000 px units) | 4.44 ± 0.29 | 4.41 ± 0.16 | 4.26 ± 0.32 | 4.91 ± 0.6 |
| Total Branching Points | 7898 ± 491 * | 7250 ± 438 | 7023 ± 668 | 8649 ± 242 |
| No. Segments | 8415 ± 458 * | 7741 ± 445 | 7629 ± 732 | 9044 ± 280 |

| | | | | |
|---|---|---|---|---|
| Data are mean ± SD; (†) p<.1 , (*) p< 0.05, (**) p< 0.01 by T-Test compared to control-untreated group. N=5 per group | | | | |

### Biomarker activity

In P14 and P18, eyes were collected, homogenized and centrifuged, to compare the levels of different biomarkers in the tissue of the different study groups. Samples included 4 different rats per group, for each 3 samples were tested. The contents were normalized to total protein concentration in each sample. The analyzed biomarkers were 8-isoprostane, or 8-isoPGF2α, a commonly studied and abundantly generated in vivo during oxidative stress and lipid peroxidation, and is a reliable and proven biomarker for oxidative stress (Beharry 2017). Additionally, PGE2 - a biomarker for inflammatory processes, which has dual opposing effects on endothelial cells was also measured (Figure 11B). It mediates both vasoconstriction and vasodilation (through different receptors). PGE2 is the principle metabolite of the COX-2 isoform which is activated by cytokines and growth factors, and is highly involved in angiogenesis (Beharry 2017).

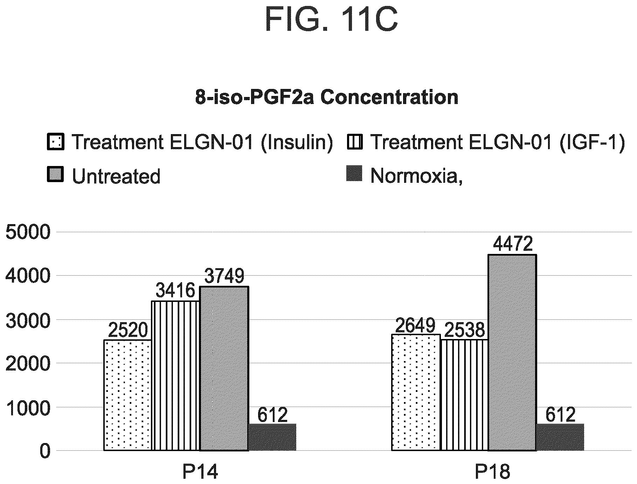

Results showed reduced 8-isoPGF2α levels in P14 and P18 compared to the untreated group, indicating a preventative effect on the oxidative stress damage, created by the animal model (Figure 11C). The effect is seen both in the first and second stage of the disease (P14 and P18). Levels of PGE2 are higher in P14 in all groups compared to Normoxia, with the treatment groups showing a decrease in P18, in which the untreated group shows a significant increase, related to the inflammatory stage of the pathology (Figure 11B).

### EXAMPLE 4

### Study 3

An ophthalmic formulation (ELGN01 including Insulin, DHA and Coq10 (described in Example 1) was administered to newborn rats to determine the concentration of insulin in the eye following administration.

### Procedure

Two rat dams with litters of 18 pups were divided into two groups: Group A - ELGN01 Normoxia group (18), Group B - ELGN01 Hypoxia group (18). A single dam with 2 pups placed under normoxia conditions was used a control.

Treatment was initiated at day 5 for 4 days by administering the composition (a dose of 10 µL that contained 0.0067 insulin units) under the eyelid using a syringe (topical). Rats were sacrificed at 30, 60, 120 minutes post administration (N=3 per T). Whole eyes were homogenized and assessed via ELISA (Quantikine^{®} ELISA).

### Results

The results shown in Table 9 below indicate that 8-16% of administered insulin was absorbed into ocular tissues within the first two hours.

**Table 9 - Averages per time-point (±SDV)**

| **Time** | **Insulin concentration (Pmol/L)** |
|---|---|
| 30 | 5.52 ± 2.05 |
| 60 | 4.66 ± 0.92 |
| 120 | 4.80 ± 1.15 |

### EXAMPLE 5

### Nanoemulsion Formulations

The following example demonstrates an alternative approach for manufacturing the composition-of-matter of the present invention.

The instant invention discloses one-pot conjugation of insulin to oily nanodroplets directly in the course of formulation process. The conjugation is performed by one-step coupling of insulin to DHA carboxyl groups in an aqueous medium using crosslinking reagent *N*-(3-Dimethylaminopropyl)-*N*'-ethylcarbodiimide hydrochloride (EDC).

The process for making an insulin-DHA conjugate utilizes the following general steps:
- Formation of nanodroplets using solvent displacement method
- Activation of DHA carboxyl group with EDC by formation of an active O-acylisourea DHA-EDC ester.
- Conjugation of insulin to the DHA carboxyl group by formation of amide bond with the primary amine groups of insulin accompanying by release of an EDC by-product as a soluble N-unsubstituted urea.
- Purification of reaction mixture from EDC by-product using ultrafiltration through 30,000-100,000 MWCO membranes

### Materials and Methods

Preparation of organic phase - 347mg DHA free acid, 75mg Tyloxapol and 75mg Lipoid E80 were dissolved in 25ml Ethanol. The mixture was added dropwise through 21G needle to 100ml of the double deionized water, continuously mixed at 350RPM at room temperature. Resulting emulsion was mixed for additional 10 minutes and afterwards the organic solvent was completely removed under reduced pressure using a laboratory rotary evaporator (40±2°C, 50mBar).

The obtained emulsion was subjected to amine coupling reaction after preliminary pH adjustment to 4.5 with 0.1N HCl.
0.27mmol of EDC dissolved in 1ml water was added to the resulting emulsion, the mixture was incubated at room temperature for 40 minutes until formation of DHA-EDC intermediate ester is completed.

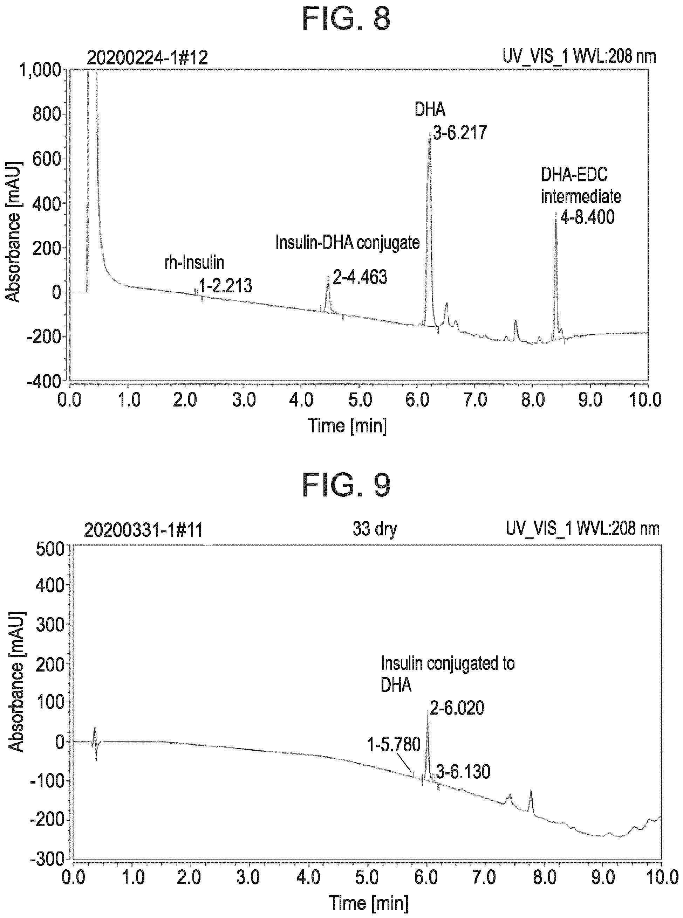

The pH of the reaction mixture was adjusted to 6.2 and 0.045mmol of insulin dissolved in 50ml water (pH 7.2) was added. The reaction was completed during 1 hour, pH 6.3-6.4 was maintained during the coupling. The reaction was monitored by HPLC (Dionex Ultimate 3000), the method conditions and chromatogram of the reaction mixture at time point 30 min are provided in Fig.8.

Upon reaction completion, the mixture was diluted 1:2 with double deionized water and transferred through 100,000 MWCO Hydrosart ultrafiltration cassette (Sartorius) using peristaltic pump.

Content of insulin conjugate in 150ml of the resulting retentate was 0.037 mmol, yield 82% calculated to insulin content.
Osmolarity of the emulsion was 301mosm/kg.

A chromatogram of the coupling reaction mixture is presented in Figure 8, the components and conditions are listed in Table 10 below.

**Table 10**

| **Method parameter** | **Setting value** |
|---|---|
| Column | Thermo, Hypersil Gold-C18 column (3 × 50mm, 3µm) |
| Column temperature | 45°C |
| Injection volume | 5µl |
| Flow rate | 1.0ml/min |
| UV detector | 208nm |
| Mobile Phase A | 1000ml Water : 1ml TFA |
| Mobile Phase B | 750ml Acetonitrile: 150ml Methanol: 100ml IPA: 0.5ml TFA |
| Gradient | 0-7.5min 30% B to 100%B |
| | 7.5-11.0 min 100%B |

Another composition-of-matter formulated as a lyophilized powder suitable for reconstitution into an oil-in-water nanoemulsion was manufactured. Table 11 below lists the components used in the manufacturing process.

**Table 11**

| Ingredient | Function |
|---|---|
| Recombinant Human Insulin (rh-Insulin) | Active substance |
| Cis-4,7,10,13,16,19- Docosahexaenoic acid (DHA) , as free acid and as ethyl ester | Active substance |
| Coenzyme Q10 | Active substance |
| Tyloxapol | Nonionic surfactant |
| Lipoid E 80 | Amphiphilic surfactant |
| Polyvinyl alcohol (PVA) | Nonionic surfactant |
| 0.1N NaOH | Reagent, pH adjustment |
| 0.1N HCl | Reagent, pH adjustment |
| Water (DDW)¹ | Aqueous phase |
| Ethanol ¹ | Solvent |
| Acetone ¹ | Solvent |
| 1-ethyl-3-(3-dimethylaminopropyl)carbodiimide hydrochloride (EDC) ^{1, 2} | Reagent |
| 2-Hydroxypropyl-β-cyclodextrin (HPbCD) | |
| | Stabilizer, cryoprotectant |

| | |
|---|---|
| ¹ Removed during the process ² Crosslinking reagent used in amine coupling reaction and removed afterwards | |

The formulation process includes preparation of two separate emulsions: a first emulsion incorporates Coenzyme Q10 into the DHA nanodroplets and a second emulsion includes insulin conjugated to the DHA nanodroplets. The emulsions were prepared separately by displacement method and combined prior to the purification step.

### Emulsion 1

300mg of Coenzyme Q10, 525mg of DHA ethyl ester, 125mg Tyloxapol and 125mg Lipoid E80 were dissolved in a mixture of 15ml Acetone and 50ml Ethanol. The mixture was added dropwise through 21G needle to 250ml of 0.1% PVA aqueous solution continuously mixed at 350RPM at room temperature. Resulting emulsion was mixed for additional 10 minutes and afterwards the organic solvents was completely removed under reduced pressure using a laboratory rotary evaporator (45±2°C, 50mBar).

### Emulsion 2

125mg of DHA free acid, 25mg Tyloxapol and 25mg Lipoid E80 were dissolved in 12ml Ethanol. The mixture was added dropwise through 21G needle to 50ml of double deionized water continuously mixed at 350RPM at room temperature. Resulting emulsion was mixed for additional 10 minutes and afterwards the organic solvents was completely removed under reduced pressure using a laboratory rotary evaporator (40±2°C, 50mBar).

The obtained emulsion was subjected to amine coupling reaction after preliminary pH adjustment to 4.5 with 0.1N HCl.

0.11mmol of EDC dissolved in 1ml water was added to the resulting emulsion, the mixture was incubated at room temperature for 1.25 hours until formation of DHA-EDC intermediate ester is completed.
pH the reaction mixture was adjusted to 6.2 and 0.015mmol of insulin dissolved in 18ml water (pH 4.2) was added. The reaction was completed during 1 hour, pH 6.2-6.4 was maintained during the coupling. The reaction was monitored by HPLC (Dionex Ultimate 3000), the method conditions and typical chromatogram of the reaction mixture are provided in Figure 8.

Upon reaction completion, the mixture was combined with Emulsion #1 and then diluted 1:2 with 0.1% PVA aqueous solution (osmolarity <5mosm/kg) and transferred through 30,000 MWCO Hydrosart ultrafiltration cassette (Sartorius) using peristaltic pump, final volume of retentate was 250ml (theoretical content of conjugated insulin was 0.06µmol/ml).

4g of HPBCD dissolved in 8ml water was added to 80ml of emulsion and the volume was adjusted to 100ml.
The emulsion was filtered through the 0.22µm PES membrane, filled into the glass vials, 4ml (0.5ml per vial) and lyophilized. Osmolarity of the finished bulk product was 376 mosm/kg.

Theoretical content of insulin conjugate per vial was 0.024µmol/vial, observed content was 0.017µmol/vial; yield of conjugated insulin was 72%.

Z-Average size of the liquid bulk and dry finished product were 119.9nm (polydispersity index 0.137) and 243nm (polydispersity index 0.342), respectively.

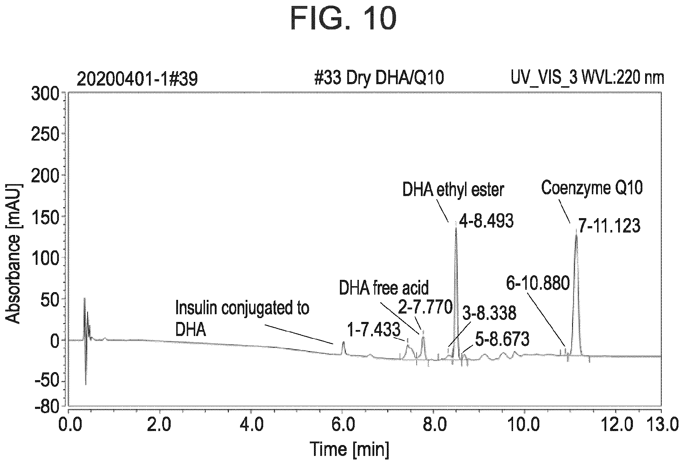

Content of conjugated insulin as well as DHA and Coenzyme Q10 the lyophilized powder are monitored by RP-HPLC. Chromatograms of the lyophilized finished product are shown in Figure 10. Table 12 below provides chromatographic conditions used for testing of the lyophilized formulation.

**Table 12**

| **Method parameter** | **Setting value** |
|---|---|
| Column | Thermo, Hypersil Gold-C18 column (3 × 50mm, 3µm) |
| Column temperature | 45°C |
| Injection volume | 5µl |
| Flow rate | 1.0ml/min |
| UV detector | 208nm for monitoring of conjugated insulin |
| | 220nm for monitoring of DHA and Coenzyme Q10 |
| Mobile Phase A | 1000ml Water : 1ml TFA |
| Mobile Phase B | 750ml Acetonitrile: 150ml Methanol: 100ml IPA: 0.5ml TFA |
| Gradient | 0-3.0min 30% B to 42%B |
| | 3.0-8.5 min 42%B to 100%B |
| | 8.5-12.5min 100% B |

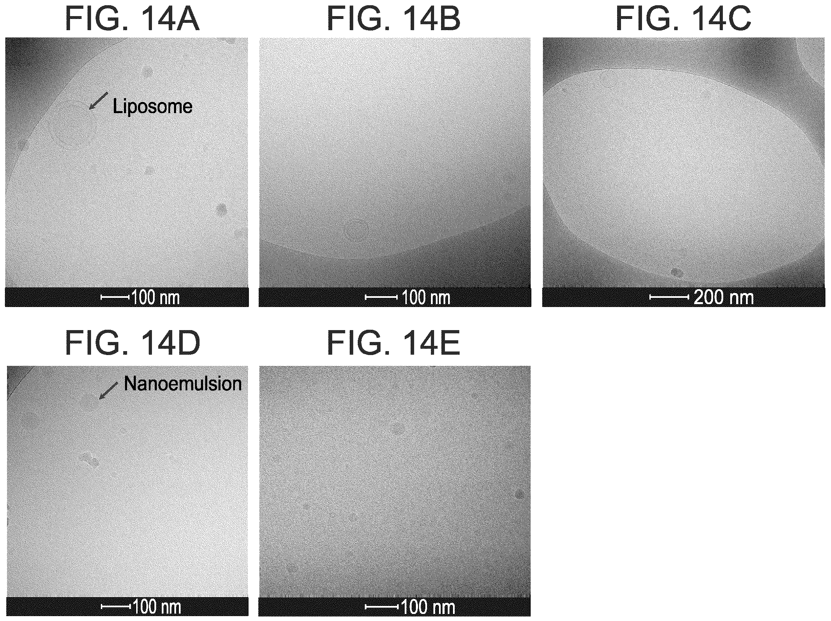

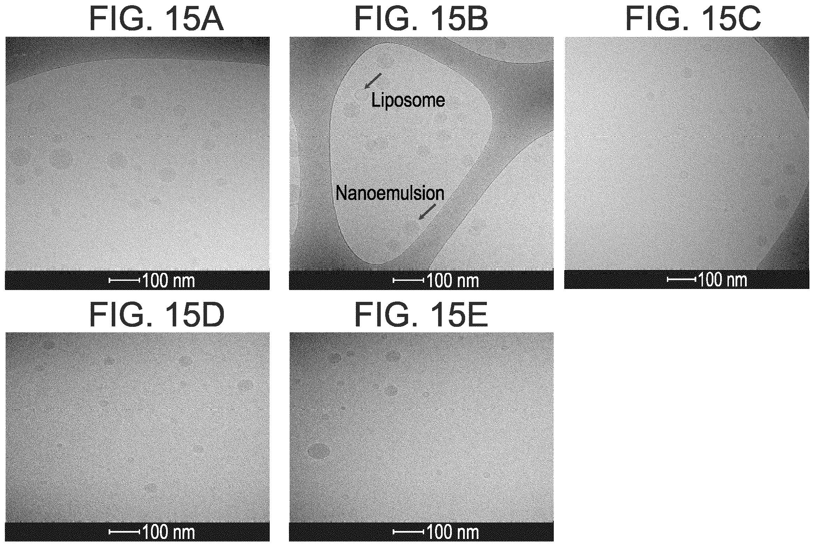

Typical Cryo transmitting electron micrographs (TEM) of the drug product produced as described in Example 5 are demonstrated in Figures 14A-E and 15A-E.

### EXAMPLE 6

### GI Formulation

An oral emulsion for local treatment of intestinal malabsorption in preterm infants was manufactured. The formulation contains three active ingredients: rh-Insulin, DHA and Coenzyme Q10. In the reconstituted formulation, insulin exists as free protein, DHA and Coenzyme Q10 are incorporated to the oil droplets.

The formulation process included the following general steps:
- Formation of DHA and Coenzyme Q10 emulsion using solvent displacement method
- Addition of rh-Insulin and cryoprotectant
- Filtration and lyophilization

### Materials and Methods

513mg of Coenzyme Q10, 898mg of DHA ethyl ester, 175mg Tyloxapol and 175mg Lipoid E80 were dissolved in 80ml Ethanol. The mixture was added dropwise through 21G needle to 350ml of 0.1% PVA aqueous solution continuously mixed at 350RPM at room temperature. Resulting emulsion was mixed for additional 10 minutes and afterwards the organic solvents was completely removed under reduced pressure using a laboratory rotary evaporator (45±2°C, 50mBar).

1ml of Insulin solution in water (2.7mg/ml, pH 8.5) was mixed with 14ml of cryoprotectants solution containing 28.6mg/ml HPBCD and 343mg/ml Maltodextrin. The obtained solution was added to continuously mixed emulsion and mixed for 20minutes.

The emulsion was filtered through 0.22um PES membrane, filled into the glass vials, 4ml (filling volume 0.5ml/vial) and lyophilized. Osmolarity of the finished bulk product was 358 mosm/kg. Each vial contains 0.65 IU rh-Insulin, 0.9mg DHA and 0.5mg Coenzyme Q10.

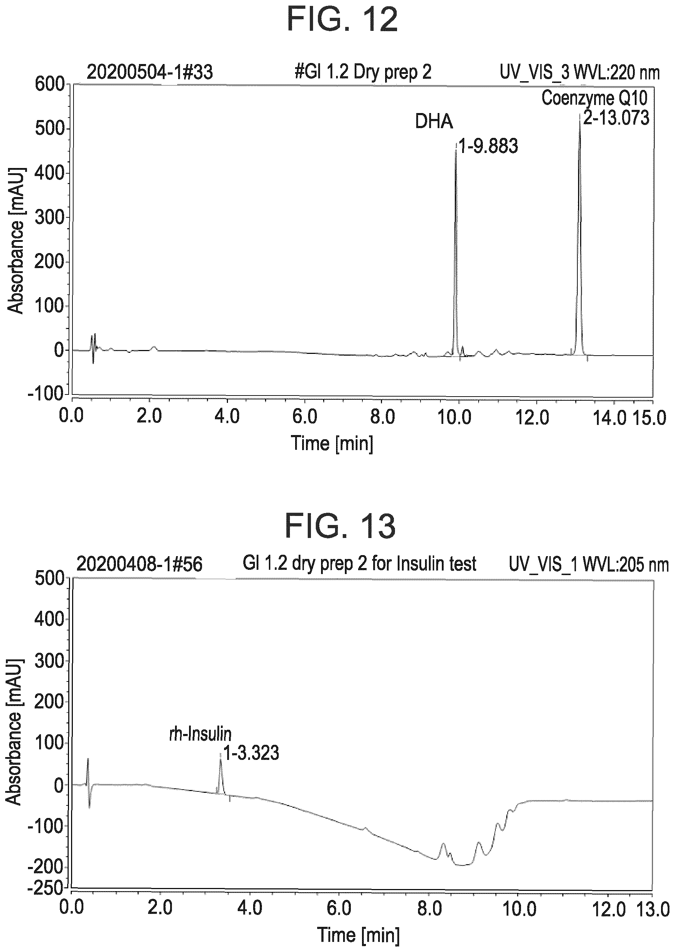

Table 13 below lists the formulations components. Chromatograms of the lyophilized product are provided in Figures 12 and 13.

**Table 13**

| **Ingredient** | **Quantity per 1ml of reconstituted product** |
|---|---|
| Recombinant Human Insulin (rh-Insulin) | 1.3 IU (0.045mg) |
| Cis-4,7,10,13,16,19- Docosahexaenoic acid (DHA) | 1.9mg |
| Coenzyme Q10 (CoQ10) | 1.1mg |
| Tyloxapol | 0.4mg |
| Lipoid E 80 | 0.4mg |
| Polyvinyl alcohol (PVA) | 0.8mg |
| Hydroxypropyl-beta-cyclodextrin (HPbCD) | 6.7mg |
| Maltodextrin | 80.0mg |
| Sodium hydroxide* | - |
| Hydrochloric acid* | - |
| Water for injection | Up to 1ml |

| | |
|---|---|
| *Sodium hydroxide or hydrochloric acid are used for adjusting the pH value and not included in the sum. | |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

In addition, any priority document(s) of this application is/are hereby incorporated herein by reference in its/their entirety.

In the following, some aspects of the invention are described:
1. A pharmaceutical composition comprising insulin, Docosahexaenoic acid (DHA) and coenzyme Q10.
2. The pharmaceutical composition of aspect 1, further comprising insulin-like growth factor (IGF).
3. The pharmaceutical composition of aspect 1, further comprising a carrier formulated for topical delivery.
4. The pharmaceutical composition of aspect 1, further comprising a carrier formulated for ocular delivery.
5. The pharmaceutical composition of aspect 4, wherein said carrier includes a surfactant.
6. The pharmaceutical composition of aspect 1, formulated as an oil-in-water nanodroplets emulsion with said insulin conjugated to said nanoparticles.
7. The pharmaceutical composition of aspect 6, wherein said insulin is amide-conjugated to said nanodroplets.
8. The pharmaceutical composition of aspect 6, wherein said nanodroplets include said Docosahexaenoic acid (DHA) and said coenzyme Q10.
9. The pharmaceutical composition of aspect 8, wherein said insulin is amide-conjugated to Docosahexaenoic acid (DHA).
10. The pharmaceutical composition of aspect 1, wherein a concentration of said insulin is 0.001U to 20U per ml.
11. The pharmaceutical composition of aspect 1, wherein a concentration of said DHA is 1-3 mg/ml.
12. The pharmaceutical composition of aspect 1, wherein a concentration of said coenzyme Q10 is 1-3 mg/ml.
13. The pharmaceutical composition of aspect 2, wherein a concentration of said IGF is 0.001U to 20U per ml.
14. A method of treating retinopathy in preterm infants comprising administering a pharmaceutical composition including insulin, Docosahexaenoic acid (DHA) and coenzyme Q10 to an eye of a preterm infant thereby treating retinopathy in preterm infants.
15. A method of prevention or reducing severity of retinopathy in preterm infants comprising administering a pharmaceutical composition including insulin, Docosahexaenoic acid (DHA) and coenzyme Q10 to an eye of a preterm infant thereby prevention or reducing severity of retinopathy in preterm infants.
16. A method of reducing retinal hemorrhages in preterm infants comprising administering a pharmaceutical composition including insulin, Docosahexaenoic acid (DHA) and coenzyme Q10 to an eye of a preterm infant thereby reducing retinal hemorrhages in preterm infants.
17. A method of reducing retinal hemorrhages in subjects experiencing retinopathy comprising administering a pharmaceutical composition including insulin, Docosahexaenoic acid (DHA) and coenzyme Q10 to an eye of a subject thereby reducing retinal hemorrhages in subject eye.
18. A method of reducing retinal neovascularization in subjects experiencing retinopathy comprising administering a pharmaceutical composition including insulin, Docosahexaenoic acid (DHA) and coenzyme Q10 to an eye of a subject thereby reducing retinal neovascularization in subject eye.
19. A method of increasing retinal vascular coverage in preterm infants comprising administering a pharmaceutical composition including insulin, Docosahexaenoic acid (DHA) and coenzyme Q10 to an eye of a preterm infant thereby increasing retinal vascular coverage in preterm infants (reducing avascular retinal areas).
20. A method of reducing retinal inflammation in preterm infants comprising administering a pharmaceutical composition including insulin, Docosahexaenoic acid (DHA) and coenzyme Q10 to an eye of a preterm infant thereby reducing retinal inflammation in preterm infants.
21. A method of reducing retinal oxidative stress in preterm infants comprising administering a pharmaceutical composition including insulin, Docosahexaenoic acid (DHA) and coenzyme Q10 to an eye of a preterm infant thereby reducing retinal oxidative stress in preterm infants.
22. A method of improving retinal layer development in preterm infants comprising administering a pharmaceutical composition including insulin, Docosahexaenoic acid (DHA) and coenzyme Q10 to an eye of a preterm infant thereby improving retinal layer development in preterm infants.
23. A method of reducing vision impairment (incidence or severity) in preterm infants comprising administering a pharmaceutical composition including insulin, Docosahexaenoic acid (DHA) and coenzyme Q10 to an eye of a preterm infant thereby reducing vision impairment in preterm infants.
24. A method of increasing visual field in preterm infants comprising administering a pharmaceutical composition including insulin, Docosahexaenoic acid (DHA) and coenzyme Q10 to an eye of a preterm infant thereby increasing visual field in preterm infants.
25. The method of any of aspects 14-24, wherein said pharmaceutical composition is an aqueous nanoemulsion.
26. The method of aspect 25, wherein said administering is effected by applying one or more drops of said pharmaceutical composition to said cornea.
27. The method of any of aspects 14-24, wherein said pharmaceutical composition is applied to said preterm infant anytime within a time period spanning birth to six months of age.
28. The method of any of aspects 14-24, wherein said administering is effected via one or more intra-vitreous injections of said pharmaceutical composition.
29. A method of formulating a pharmaceutical composition for topical treatment of retinopathy comprising:
   (a) generating an oil-in-water nanoemulsion including Docosahexaenoic acid (DHA) and Coenzyme Q10 in the oil phase; and
   (b) conjugating Insulin or IGF-1 to nanodroplets of said nanoemulsion using an amine coupling reaction or providing said Insulin in its free form.
30. The method of aspect 29, further comprising (c) purifying nanodroplets conjugated to said Insulin or IGF-1.
31. The method of aspect 20, further comprising (d) adding a stabilizing agent following (c).
32. The method of aspect 31, wherein said stabilizing agent is cyclodextrin.
33. The method of aspect 31, further comprising lyophilization following (d).

## Claims

1. A pharmaceutical composition comprising an oil-in-water nanodroplets emulsion formulated for topical delivery, said emulsion including Insulin-like growth factor 1 (IGF-1) amide-conjugated to Docosahexaenoic acid (DHA) and coenzyme Q10.

2. The pharmaceutical composition of claim 1, further comprising a surfactant.

3. The pharmaceutical composition of claim 1, wherein a concentration of said IGF-1 is 0.001U to 20U per ml.

4. The pharmaceutical composition of claim 1, wherein a concentration of said DHA is 1-3 mg/ml.

5. The pharmaceutical composition of claim 1, wherein a concentration of said coenzyme Q10 is 1-3 mg/ml.

6. The pharmaceutical composition of claim 5, wherein said Docosahexaenoic acid (DHA) is conjugated to a primary amine group of IGF-1.
